# EUROPÄISCHE PATENTSCHRIFT

(11) **EP 2 701 622 B1**
(45) Veröffentlichungstag und Bekanntmachung des Hinweises auf die Patenterteilung: **04.03.2015**
(21) Anmeldenummer: 12766903.4
(22) Anmeldetag: 27.04.2012
(51) Int. Cl.: A61B 17/70, A61B 17/86, A61B 19/00

(54) **VERSCHLUSSEINRICHTUNG FÜR KANÜLIERTE KNOCHENSCHRAUBEN**
CLOSURE APPARATUS FOR CANNULATED BONE SCREWS
DISPOSITIF DE FERMETURE POUR VIS D'OSTÉOSYNTHÈSE CANULÉES

(30) Priorität: 27.04.2011 DE 102011017602
(43) Veröffentlichungstag der Anmeldung: 05.03.2014
(60) Teilanmeldung: 15150799.3
(73) Patentinhaber: Silony Medical International AG, 8500 Frauenfeld (CH)
(72) Erfinder: TRAUTWEIN, Frank, 70794 Filderstadt (DE); HEUER, Frank, 70794 Filderstadt (DE); CLAUS, Frank, 55270 Essenheim (DE); BERNHARD, Andreas, CH-3065 Bolligen (CH)
(74) Vertreter: DREISS Patentanwälte PartG mbB
(86) Internationale Anmeldenummer: PCT/EP2012/057820
(87) Internationale Veröffentlichungsnummer: WO 2012/146744

(56) Entgegenhaltungen:
- EP-A1- 2 140 824
- WO-A1-2006/108329
- WO-A1-2009/010247
- US-A1- 2007 038 219
- US-A1- 2007 293 866
- US-A1- 2010 280 558

## Beschreibung

Die Erfindung betrifft eine Einrichtung zum einseitigen Verschluss einer kanülierter Öffnung in Knochenschrauben, und zwar ein Implantatsystem nach dem Oberbegriff des Anspruchs 1.

### Stand der Technik

Osteoporose ist durch eine Abnahme der strukturellen Integrität des Knochens gekennzeichnet. Häufig resultieren daraus Frakturen, welche zum großen Teil die Wirbelsäule betreffen. Aufgrund der reduzierten Knochendichte besteht die klinische Herausforderung in der unzureichenden Verankerungsstabilität bei der Versorgung mit Knochenschrauben.

Aus DE3508759A1, 1984, ist eine Knochenschraube für die Versorgung von Femurfrakturen bekannt. Die Erfindung beschreibt eine Knochenschraube mit einer mittig angeordneten sowie mehrere dazu querverlaufende Öffnungen. Durch diese Öffnungen kann Knochenzement in den Knochen injiziert werden. Dadurch wird eine höhere Festigkeit zwischen Knochenschraube und Knochen erreicht.

Schrauben mir Queröffnungen für die zusätzliche Augmentation mit Hilfe einer viskösen Adhäsionslösung sind bereits auch aus dem nicht-medizintechnischen Bereichen bekannt (US4860513A, 1988).

Aus WO0126568A1, 1999; WO2006070961A2, 2004, sind Knochenschrauben zur Stabilisierung der Wirbelsäule bekannt, welche eine vergleichbare Anordnung von Öffnungen für die Augmentation mit Knochenzement besitzen. Diese vorgestellten Schrauben sind am distalen Ende verschlossen, so dass ein extravertebraler Zementaustritt am distalen Schraubenende verhindert wird.

Der derzeitige Trend der medizinischen Versorgung geht in Richtung minimal-invasive Chirurgie. Eine Anforderung dieser Technik besteht in der Verwendung von geeigneten Zieldrähten mit denen die Position und Orientierung von Knochenschrauben vordefiniert werden kann. Deshalb müssen die Knochenschrauben eine zentrale Durchgangsöffnung, auch Kanülierung genannt, aufweisen, um der minimal-invasiven Technik gerecht zu werden. Werden zementierbare Knochenschrauben mit einer Durchgangsbohrung verwendet, besteht allerdings die Gefahr, dass bei einer bikortikalen Verschraubung sich der Knochenzement außerhalb vom Knochen verteilen kann.

Aus WO0028907A1, 1998, ist eine kanülierte Schraube bekannt, welche einen distalen Öffnungsverschluss aufweist. Die genaue Applizierung des Verschlusses ist unbekannt und in der dargestellten Ausführung klinisch nicht oder nur schwer anzuwenden.

Aus US2010030135A1, 2006; EP2140824A1, 2008, sind Anmeldungen bekannt, welche eine Applikation eines distalen Verschlusses nach dem Verschrauben im Knochen vorschlagen. Wie beispielsweise in EP2140824A1, 2008, beschrieben soll dazu ein Applikator bzw. ein Applikationsinstrument verwendet werden, welches aufgrund der vorgeschlagenen Ausgestaltung relativ aufwendig zu realisieren ist.

WO 2009/010247 A1 offenbart ein Implantatsystem nach dem Oberbegriff des Anspruchs 1, wobei das Applikationsinstrument als in die Knochenschraube eingefügter Polymerstift ausgebildet ist, der an seinem Ende den Stopfen hält und der unter Ultraschallbeaufschlagung schmilzt.

WO 2006/108329 A1 offenbart eine Knochenschraube, wobei zur nietartigen Verbindung zweier Teile, in Form einer Platte und eines Knochens, die durch beide Teile hindurchgesteckte Knochenschraube am distalen hindurchgesteckten und vorstehenden Ende aufweitbar ist, indem ein Applikationsinstrument mit ablösbarem Kopf eingesteckt und durch den Kopf das distale Ende der Knochenschraube aufgeweitet wird.

### Aufgabe

Die Aufgabe der vorliegenden Erfindung besteht darin, eine Verschlusseinrichtung für zementierbare Knochenschrauben zur Verfügung zu stellen, welche besonders für die Verwendung von Führungsdrähten und damit für die minimal-invasive Technik geeignet ist. Hierbei ist das Problem zu lösen, dass Knochenzement unbeabsichtigt durch die zentrale Durchgangsbohrung der Knochenschraube austritt. Das erfindungsgemäße Implantat bzw. Implantatsystem sollte kostengünstig in der Herstellung sein da es sich regelmäßig um ein Instrument bzw. Implantat zum Einmalgebrauch handelt. Ferner soll die intra-operative Handhabung einfach sein.

### Lösung

Die Aufgabe wird erfindungsgemäß durch ein Implantatsystem mit den Merkmalen des Anspruchs 1 gelöst.

### Vorteile

Vorteilhaft bei der vorliegenden Erfindung ist die Reduktion des Herstellungsaufwands bzw. die Vereinfachung in der Handhabung. Mit der erfindungsgemäßen Verschlusseinrichtung kann die Zementapplikation bei Knochenschrauben sicherer und vor allem einfacher gestaltet werden.

### Figurenbeschreibung

Abbildung 1 zeigt einen Pedikelschraubenschaft als Stellvertreter für kanülierte Knochenschrauben.
Abbildung 2 stellt ein Applikationsinstrument mit der Verschlusseinrichtung dar.
Abbildung 3 zeigt die Applikation der Verschlusseinrichtung.

### Bevorzugte Details und Ausführungsformen

Die technischen Lösungen sind nachfolgend oft beispielhaft beschrieben. Dies soll als Mittel zur Erläuterung des zugrundeliegenden Gedankens aufgefasst und nicht als auf die jeweilige konkrete Darstellung beschränkt verstanden werden.

Bei einer nicht dargestellten erfindungsgemäßen Ausführungsform sind bei dem Applikationsinstrument mehrere Verschlusseinrichtungen (20), d.h. Stopfen (20) und Sollbruchstellen (21) vorgesehen, die gemäß Patentanspruch 1 ausgebildet sind.

Die erfindungsgemäße Verschlusseinrichtung soll generell bei Knochenschrauben Ihre Anwendung finden.

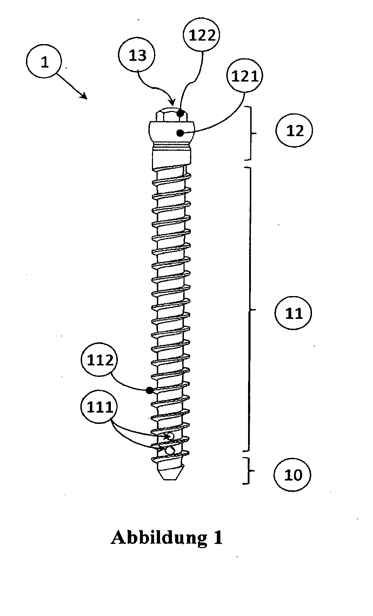

In der weiteren Beschreibung wird ein Pedikelschraubenschaft (1) als Beispiel für die unterschiedlichen Ausführungsformen der Verschlusseinrichtung dienen (Abbildung 1). Der Pedikelschraubenschaft (1) besteht aus einer distalen (10), einer mittleren (11) und einer proximalen Komponente (12), wobei an der proximalen Komponente ein Kopfteil (121) befestigt ist, welches dazu dient die Schraube mit einer dorsalen Implantatkomponente zu verbinden, z.B. einem Stab. Auf die Funktion und Ausgestaltung des Schraubenschaftkopfes (12) wird in der folgenden Beschreibung nicht näher eingegangen. Ein wesentliches Merkmal des Pedikelschraubenschafts (1) ist eine zentrale Durchgangsöffnung (13). Zu dieser Durchgangsöffnung befinden sich radial angeordnete Öffnungen (111), die mit der zentralen Durchgangsöffnung (13) kommunizieren. Des Weiteren besitzt der Pedikelschraubeschaft (1) ein Knochengewinde (112).

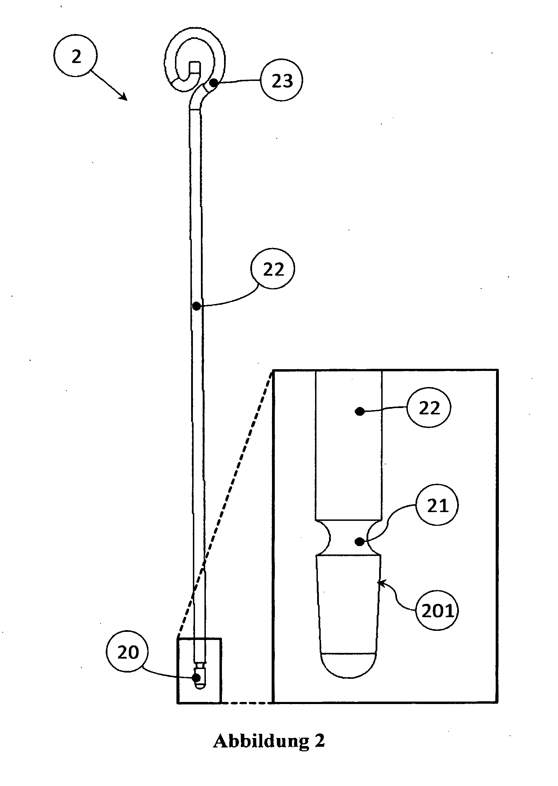

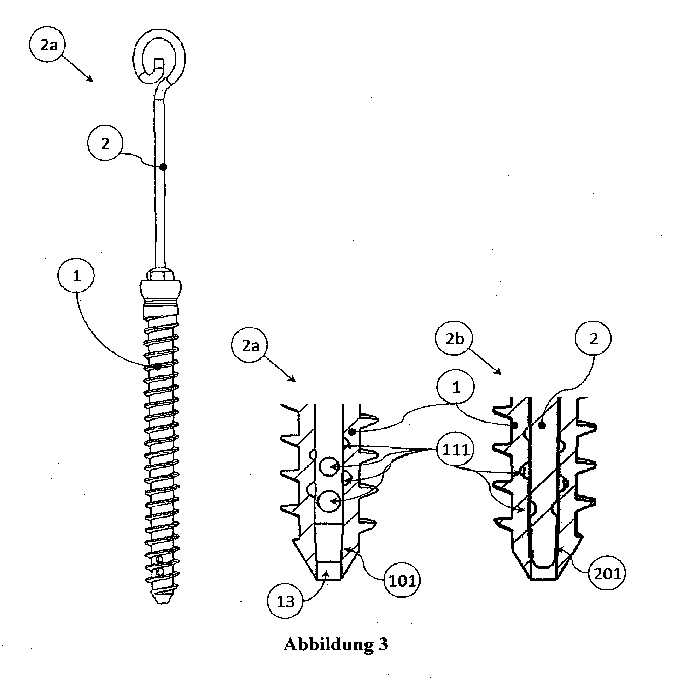

In einer ersten Ausführung befindet sich die Verschlusseinrichtung (Stopfen) (20) am distalen Ende eines Applikationsinstruments (2) (Abbildung 2). Das Applikationsinstrument besteht aus wenigstens einem Griff (23) und einem Schaft (22). Die Verschlusseinrichtung (20) ist in der ersten Ausführung als Zapfen mit mindestens einem nicht-zylindrischen Oberflächenanteil (201) ausgeführt, z.B. als Kegel mit flacher oder bauchiger Mantelfläche. Mindestens eine Verschlusseinrichtung (20) ist über wenigstens einer Sollbruchstelle (21) mit dem Applikationsinstrument (2) verbunden, so dass das Applikationsinstrument (2) zusammen mit der Verschlusseinrichtung (20) als ein Bauteil gefertigt werden kann. In dieser Ausführungsvariante weist der Pedikelschraubenschaft eine Querschnittverjüngung (101) am distalen Ende auf (Abbildung 3). Bei der Anwendung wird zuerst die Knochenschraube (1) im Knochen implantiert und geschraubt (Abbildung 3). Anschließend wird das Applikationsinstrument (2) in die zentrale Durchgangsöffnung (13) geführt (2a). Dabei wird das Applikationsinstrument (2) soweit eingeführt bis ein Kontakt der Verschlusseinrichtung (20) mit dem Pedikelschraubenschaft (1) erzeugt wird. Anschließend wird unter axialem Druck auf die Verschlusseinrichtung (20) eine zusätzliche Drehbewegung mit dem Griff (23) beaufschlagt. Aufgrund des Kegelwinkels stellt sich eine hohe Reibung zwischen der Verschlusseinrichtung (20) und der Querschnittverjüngung (101) ein, die es erlaubt dass der Stopfen (20) vom Schaft (22) abgedreht werden kann. Eine Sollbruchstelle (21) definiert dabei Bruchstelle.

In einer hier nicht gezeigten weiteren Ausgestaltungsform kann die Verschlusseinrichtung eine axiale Außenverzahnung und der Pedikelschraubenschaft (1) eine dazu kongruente axiale Innenverzahnung besitzen, so dass die Verschlusseinrichtung in den Pedikelschraubenschaft verzahnt eingebracht werden kann. Die Sollbruchstelle der Verschlusseinrichtung definiert die Bruchstelle nach Erreichen des Abdrehmoments.

Als alternative Ausführungsform zur axialen Innen- bzw. Aussenverzahnung kann eine radiale Verzahnung wie z.B. ein Außengewinde an der Verschlusseinrichtung und ein Innengewinde in der Durchgangsbohrung des Schraubenschafts vorgesehen werden, um eine Verbindung zwischen der Verschlusseinrichtung und dem Pedikelschraubenschaft zu erzeugen.

## Patentansprüche

1. Implantatsystem, bestehend aus einer Knochenschraube (1) mit einer zentralen Durchgangsöffnung (13), mindestens einer dazu radial verlaufenden und mit der zentralen Durchgangsöffnung (13) kommunizieren den Öffnung (111), und einem Applikationsinstrument mit mindestens einem Stopfen (20), wobei
a. die zentrale Durchgangsöffnung (13) am distalen Schraubenende eine Durchmesserverjüngung beinhaltet,
b. der kleinste Durchmesser der Verjüngung kleiner ist als der größte Durchmesser des mindestens einen Stopfens (20),
c. der größte Durchmesser des mindestens einen Stopfens (20) kleiner ist als der Durchmesser der zentralen Durchgangsöffnung (13) der Knochenschraube
d. dass der mindestens eine Stopfen (20) kegelförmig ausgeführt ist, **dadurch gekennzeichnet,**
e. **dass** der mindestens eine Stopfen (20) in ein Applikationsinstrument (2) integriert ist und
f. das Applikationsinstrument (2) aus mehreren Stopfen (20), einem Schaft (22) und einem Handgriff (23) besteht und dass die Stopfen (20) über Sollbruchstellen (21) mit dem Applikationsinstrument (2) verbunden sind, welche nach Erreichen des Bruch-Drehmoments die Stopfen (20) vom Applikationsinstrument (2) trennen.

2. Implantatsystem gemäß Anspruch 1, **dadurch gekennzeichnet dass** der Kegelwinkel des Stopfens zwischen 1° und 15° beträgt.

3. Implantatsystem gemäß Ansprüchen 1 oder 2, **dadurch gekennzeichnet, dass** die Sollbruchstellen (21) so angeordnet und hinsichtlich des zum Bruch erforderlichen Drehmoments für jede Sollbruchstelle so dimensioniert sind, dass das zum Bruch erforderliche Drehmoment für jede Sollbruchstelle (21) in Richtung des Handgriffs (23) ansteigt.

4. Implantatsystem gemäß einem der vorherigen Ansprüche, **dadurch gekennzeichnet, dass** sich an den Stopfen (20) und am distalen Ende der zentralen Durchgangsöffnung (13) der Knochenschraube (1) jeweils ein gegen Verdrehung wirksamer Formschluss befindet.

5. Implantatsystem gemäß einem der vorherigen Ansprüche, **dadurch gekennzeichnet, dass** sich an den Stopfen (20) und am distalen Ende der zentralen Durchgangsöffnung der Knochenschraube jeweils ein Gewinde befindet.

6. Applikationsinstrument (2) aus mehreren Stopfen (20), einem Schaft (11) und einem Handgriff (23) zur Verwendung bei einem Implantatsystem gemäß einem der vorherigen Ansprüche, **dadurch gekennzeichnet, dass** die Stopfen (20) über Sollbruchstellen (21) mit dem Applikationsinstrument (2) verbunden sind, welche nach Erreichen des Bruch-Drehmoments den Stopfen (20) vom Applikationsinstrument (2) trennen.

7. Applikationsinstrument (2) nach Anspruch 6, **dadurch gekennzeichnet, dass** die Sollbruchstellen (21) so angeordnet und hinsichtlich des zum Bruch erforderlichen Drehmoments für jede Sollbruchstelle so dimensioniert sind, dass das zum Bruch erforderliche Drehmoment für jede Sollbruchstelle in Richtung des Handgriffs (23) ansteigt.

## Claims

1. Implant system comprising a bone screw (1) with a central passage opening (13), at least one opening (111) running radially thereto and communicating with the central passage opening (13), and an application instrument with at least one plug (20), wherein
a. the central passage opening (13) contains a diameter constriction at the distal screw end,
b. the smallest diameter of the constriction is smaller than the largest diameter of the at least one plug (20),
c. the greatest diameter of the at least one plug (20) is smaller than the diameter of the central passage opening (13) of the bone screw,
d. the at least one plug (20) is formed conical, **characterized in that**
e. the at least one plug (20) is integrated in an application instrument (2), and
f. the application instrument (2) comprises several plugs (20), a shaft (22) and a handle (23), and that the plugs (20) are connected to the application instrument (2) via nominal break points (21) which break from the application instrument (2) after reaching the break torque of the plugs (20).

2. Implant system according to claim 1, **characterized in that** the cone angle of the plug is between 1° and 15°.

3. Implant system according to claim 1 or 2, **characterized in that** the nominal break points (21) are arranged and dimensioned with regard to the torque necessary for breakage for each nominal break point such that the torque necessary for breakage for each nominal break point (21) increases in the direction of the handle (23).

4. Implant system according to any of the preceding claims, **characterized in that** a form-fit connection effective against twisting is present at the plugs (20) and at the distal end of the central passage opening (13) of the bone screw (1).

5. Implant system according to any of the preceding claims, **characterized in that** a thread is present at the plugs (20) and at the distal end of the central passage opening of the bone screw.

6. Application instrument (2) comprising several plugs (20), a shaft (11) and a handle (23) for use in an implant system according to any of the preceding claims, **characterized in that** the plugs (20) are connected to the application instrument (2) via nominal break points (21) which break from the application instrument (2) after reaching the break torque of the plugs (20).

7. Application instrument (2) according to claim 6, **characterized in that** the nominal break points (21) are arranged and dimensioned with regard to the torque necessary for breakage for each nominal break point such that the torque necessary for breakage for each nominal break point increases in the direction of the handle (23).

## Revendications

1. Système d'implant, constitué par une vis d'ostéosynthèse (1) avec un orifice de passage central (13), au moins un orifice (111) orienté radialement à celui-ci et communiquant avec l'orifice de passage central (13), et par un instrument d'application avec au moins un bouchon (20), dans lequel
a. l'orifice de passage central (13) comporte un rétrécissement de diamètre au niveau de l'extrémité distale de la vis,
b. le plus petit diamètre du rétrécissement est inférieur au plus grand diamètre dudit au moins un bouchon (20),
c. le plus grand diamètre dudit au moins un bouchon (20) est inférieur au diamètre de l'orifice de passage central (13) de la vis d'ostéosynthèse,
d. ledit au moins un bouchon (20) est réalisé en forme de cône,
**caractérisé en ce que**
e. ledit au moins un bouchon (20) est intégré dans un instrument d'application (2), et
f. ledit instrument d'application (2) est constitué de plusieurs bouchons (20), d'une tige (22) et d'une poignée (23), et **en ce que** les bouchons (20) sont reliés à l'instrument d'application (2) par des points destinés à la rupture (21), par lesquels les bouchons (20) sont séparés de l'instrument d'application (2) lorsque le couple de rotation entraînant la rupture est atteint.

2. Système d'implant selon la revendication 1, **caractérisé en ce que** l'angle de cône du bouchon mesure entre 1° et 15°.

3. Système d'implant selon la revendication 1 ou 2, **caractérisé en ce que** les points destinés à la rupture (21) sont dimensionnés par rapport au couple de rotation requis pour la rupture de chaque point destiné à la rupture et sont disposés de telle sorte que le couple de rotation requis pour la rupture de chaque point destiné à la rupture (21) augmente en direction de la poignée (23).

4. Système d'implant selon l'une quelconque des revendications précédentes, **caractérisé en ce qu'**un assemblage par conjugaison de forme à action anti-rotative, est situé respectivement au niveau des bouchons (20) et au niveau de l'extrémité distale de l'orifice de passage central (13) de la vis d'ostéosynthèse (1).

5. Système d'implant selon l'une quelconque des revendications précédentes, **caractérisé en ce qu'**un filetage est situé respectivement au niveau des bouchons (20) et au niveau de l'extrémité distale de l'orifice de passage central de la vis d'ostéosynthèse.

6. Instrument d'application (2) constitué de plusieurs bouchons (20), d'une tige (11) et d'une poignée (23), destiné à être utilisé dans un système d'implant selon l'une quelconque des revendications précédentes, **caractérisé en ce que** les bouchons (20) sont reliés à l'instrument d'application (2) par des points destinés à la rupture (21), par lesquels les bouchons (20) sont séparés de l'instrument d'application (2) lorsque le couple de rotation entraînant la rupture est atteint.

7. Instrument d'application (2) selon la revendication 6, **caractérisé en ce que** les points destinés à la rupture (21) sont dimensionnés par rapport au couple de rotation requis pour la rupture de chaque point destiné à la rupture et sont disposés de telle sorte que le couple de rotation requis pour la rupture de chaque point destiné à la rupture (21) augmente en direction de la poignée (23).
